# EUROPEAN PATENT SPECIFICATION

(11) **EP 1 044 973 B1**
(45) Date of publication and mention of the grant of the patent: **08.10.2003**
(21) Application number: 00302887.5
(22) Date of filing: 05.04.2000
(51) Int. Cl.: C07D 335/06, C07D 333/54, C07D 409/06

(54) **Process for the dehalogenation of thiochroman and dihydrobenzothiophene derivatives**
Verfahren zur Herstellung von Thiochroman und Dihydrobenzothiophen-Derivaten
Procédé de préparation de dérivés de thiochromane et dihydrobenzothiophène

(30) Priority: 06.04.1999 US 286867
(43) Date of publication of application: 18.10.2000
(73) Proprietor: American Cyanamid Company, Madison, New Jersey 07940-0874 (US); IDEMITSU KOSAN CO., LTD., Tokyo 100-8321 (JP)
(72) Inventor: Drabb, Thomas Walter Jr., Trenton, NJ 08611 (US)
(74) Representative: Langfinger, Klaus Dieter, Dr.

(56) References cited:
- US-A- 5 519 150
- US-A- 5 607 898
- M.W. BREDENKAMP ET AL.: "Crystal Structures and Conformational Analysis of Ochratoxin A and B: Probing the Chemical Structure causing Toxicity" JOURNAL OF THE CHEMICAL SOCIETY, PERKIN TRANSACTIONS 2, 1989, pages 1835-1839, XP002152259 LETCHWORTH GB

## Description

### BACKGROUND OF THE INVENTION

Thiochroman and dihydrobenzothiophene herbicidal agents and methods of their preparation are described in U.S. 5,506,194, U.S. 5,607,898 and WO 97/08164 among other publications. The 6-(arylcarbonyl)thiochroman and 5-(arylcarbonyl)dihydrobenzothiophene derivatives are effective herbicidal agents at low rates of application and demonstrate selective control of noxious weeds in the presence of important economic crops such as corn and rice.

A common route to prepare these useful herbicidal agents entails the dehalogenation of an 8-halo-6-carbethoxythiochroman or 7-halo-5-carbethoxydihydrobenzothiophene intermediate compound. Heretofore, methods to dehalogenate said intermediate compound required high pressure hydrogenation techniques. Further, product yield and quality may be less than satisfactory. The importance of the dehalogenated 6-carbethoxythiochroman and 5-carbethoxydihydrobenzothiophene intermediates, particularly as key intermediates in the manufacture of herbicidal arylcarbonylthiochroman and arylcarbonyldihydrobenzothiophene agents, creates a significant need in the art for alternative and effective processes for their preparation.

### SUMMARY OF THE INVENTION

The present invention provides a safe efficient process for the preparation of a compound of formula I 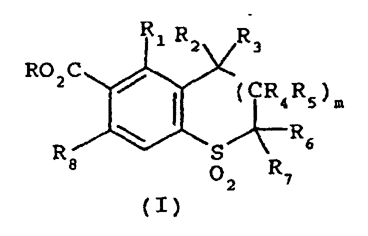 wherein
R and R₁ are each independently H or C₁-C₄alkyl;
R₂ and R₃ are each independently H, C₁-C₄alkyl or R₂ and R₃ may be taken together with the atom to which they are attached to form a group C=NOR₉ or C=O;
R₄, R₅, R₆, R₇, R₈ and R₉ are each independently H or C₁-C₄alkyl; and
m is 0 or 1
which process comprises reacting a compound of formula II 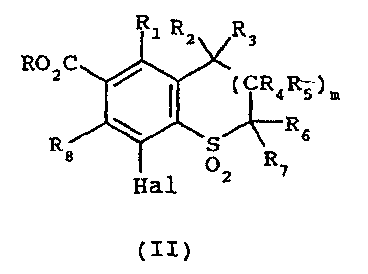 wherein Hal is Cl, Br or I and R, R₁, R₂, R₃, R₄, R₅, R₆, R₇, R₈ and m are as described hereinabove with a catalytic amount of Pd/C and at least two molar equivalents of ammonium formate in the presence of a polar solvent optionally at an elevated temperature.

### DETAILED DESCRIPTION OF THE INVENTION

Compounds of formula I are important key intermediates in the manufacture of thiochroman and dihydrobenzothiophene herbicidal agents. Methods to prepare the formula I intermediates commonly require high pressure hydrogenation procedures which may give less than satisfactory product yield and quality. Surprisingly, it has now been found that compounds of formula I may be readily and efficiently prepared from their halo precursors at atmospheric pressure using a transition metal catalyst and ammonium formate as a hydrogen source in the presence of a polar solvent, optionally at an elevated temperature. Advantageously, the product formula I compounds are obtained in good yield and high quality. The reaction is shown in flow diagram I.

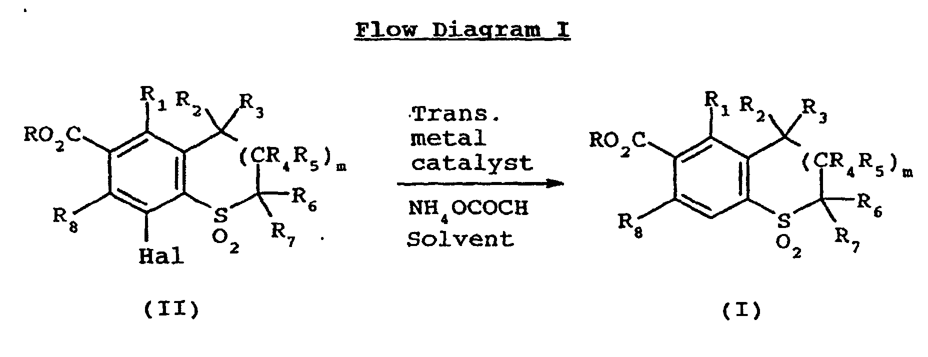

Polar solvents suitable for use in the process of the invention include alkanols, carboxylic acids or mixtures thereof, preferably methanol, ethanol or a mixture thereof.

Transition metal catalysts suitable for use in the inventive process include those transition metals commonly used in catalytic hydrogenation procedures such as. Pt, Pd, Ni, Rh and similar conventional transition metals, preferably Pd. A catalytic amount designates that amount of catalyst required to facilitate the reaction and may range from an equimolar amount to a trace amount, preferably about 5 mole% to 20 mole%.

Suitable reaction temperatures useful in the inventive process may range from ambient temperatures to the reflux temperature of the solvent or solvent mixture. In general, increased reaction temperature leads to increased reaction rate and drives the reaction to completion. However, excessively high temperatures may be detrimental and are not required. Preferable reaction temperatures are about 25°C-200°C, more preferably about 50°C-150°C.

Stoichiometrically, the inventive process requires at least 2 molar equivalents of ammonium formate in order to go to completion, however excess amounts of ammonium formate may be employed without detrimental effect.

Therefore, in accordance with the process of the invention, a compound of formula II, preferably wherein Hal is chlorine, is admixed with a polar solvent, preferably an alkanol, acetic acid or a mixture thereof, treated with at least 2 molar equivalents of ammonium formate, preferably 3 to 5 molar equivalents, and a catalytic amount of a transition metal catalyst, preferably Pd on carbon (Pd/C) at temperatures ranging from ambient temperatures to the reflux temperature of the solvent, preferably about 25°C - 200°C, more preferably about 50°C - 150°C, to obtain the desired formula I compound.

Compounds of formula II preferred for use in the inventive process include those compounds of formula II wherein Hal is Cl; m is 1; and R₂ and R₃ are taken together with the atom to which they are attached to represent C=NOR₉. Another group of formula II compounds preferred for use in the inventive process are those compounds wherein Hal is Cl; m is 0; and R₂ and R₃ are each independently C₁-C₄alkyl.

Thiochroman and dihydrobenzothiophene herbicidal agents may be prepared from compounds of formula II via the inventive process to form the intermediate compound of formula I; when R is C₁-C₄alkyl, hydrolyzing the formula I compound to the corresponding carboxylic acid of formula III; and reacting the formula III acid with an hydroxypyrazolyl compound of formula IV to obtain the arylcarbonyl compound of formula V, optionally reacting the formula V compound with a sulfonyl chloride of formula VI to obtain the arylcarbonyl compound of formula VII. The formula V and formula VII arylcarbonylthiochromans and dihydrobenzothiophenes are potent herbicidal agents. The reaction sequence is shown in flow diagram II wherein R₁₀ is C₃-C₄alkyl; R₁₁ is H or C₁-C₄alkyl; and R₁₂ is C₁-C₆alkyl or phenyl optionally substituted with one to three halogen, NO₂, C₁-C₄alkyl, or C₁-C₄alkoxy groups.

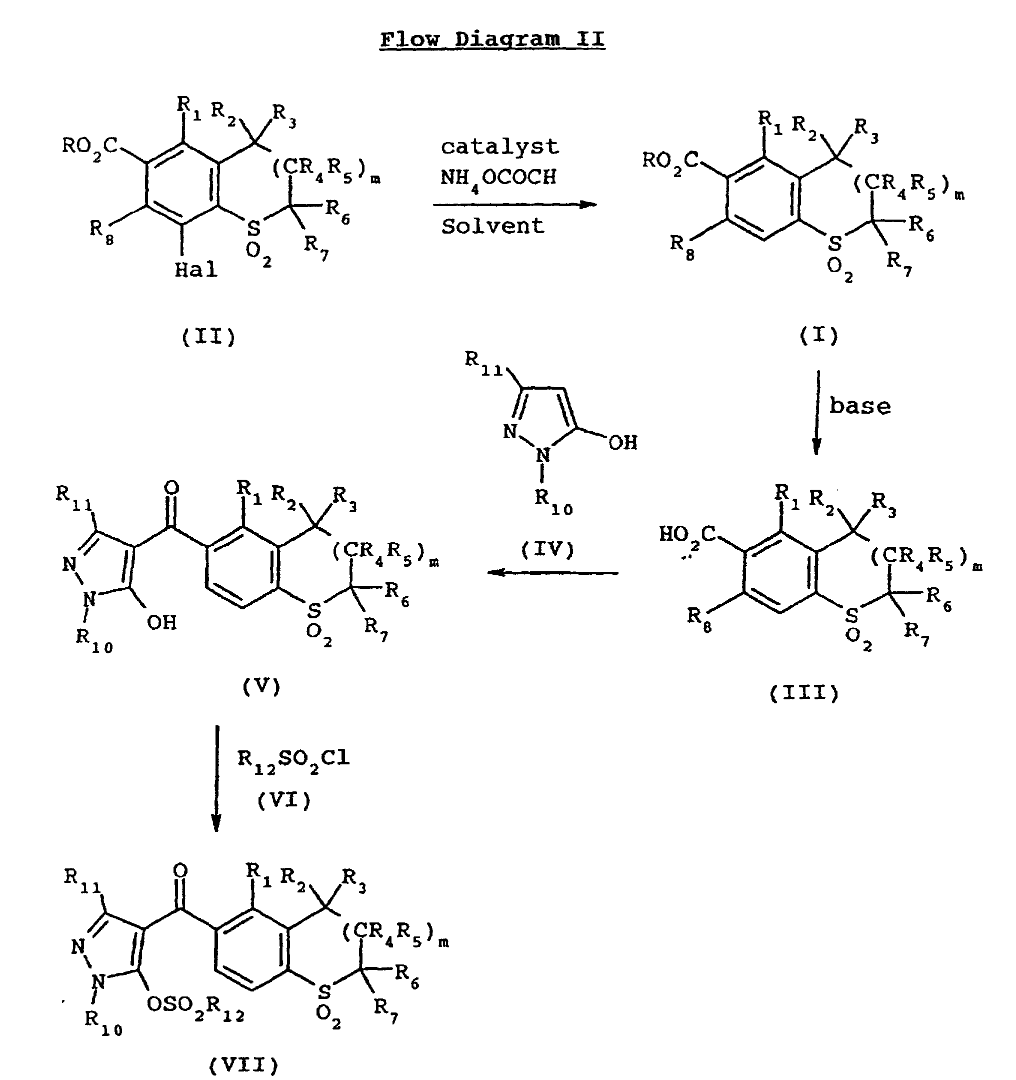

The herbicidal agents of formula V and formula VII and their preparation from compounds of formula I are described in U.S. patents 5,506,194 and 5,607,898. The hydrolysis and sulfonation steps described above may be accomplished using conventional methods. The coupling of the hydroxypyrazole compound of formula IV and the subsequent rearrangement to the desired compound of formula V may be performed using standard methods such as those described in U.S. 5,506,194 and U.S. 5,607,898, i.e. in the presence of a base and a dehydrating agent.

For a more clear understanding of the invention, the following examples are set forth below. These examples are merely illustrative and are not understood to limit the scope of underlying principles of the invention in any way. Indeed, various modifications of the invention, in addition to those shown and described herein, will become apparent to those skilled in the art from the following examples and the foregoing description. Such modifications are also intended to fall within the scope of the appended claims.

The terms ¹HNMR and ¹³CNMR designate proton and carbon 13 nuclear magnetic resonance, respectively. The term IR designates infrared spectroscopy. HPLC designates high performance liquid chromatography.

### EXAMPLE 1

### Preparation of Ethyl 2,3-Dihydro-5-methyl-4-oxo-4H-1-benothiopyran-6-carboxylate, 1,1-dioxide, 4-0-methoxyloxime

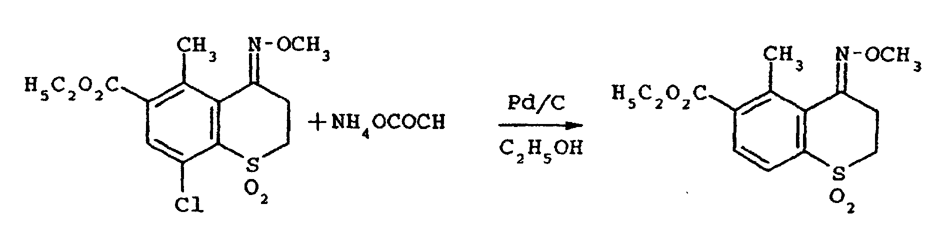

A stirred mixture of ethyl 8-chloro-2,3-dihydro-5-methyl-4-oxo-4H-1-benzothiopyran-6-carboxylate, 1,1-dioxide, 4-(0-methyloxime) (800g, 2.31 mol) in ethanol is treated with ammonium formate (729.4g, 11.57 mol) and with 10% Pd/C (50% water) (800g, 0.375 mol), heated to reflux temperature over a 1 hour period, held at reflux temperature for about 4 hours (until reaction is complete by HPLC analysis), stirred at ambient temperatures for 19 hours and filtered through a bed of diatomaceous earth. The filtercake is slurried in tetrahydrofuran (THF) and filtered several times. All filtrates are combined, concentrated to about 1/3 the original volume and filtered though a bed of diatomaceous earth. The filtercake is washed with additional THF. The filtrates are combined, labelled filtrate A and set aside.

The above procedure is repeated using the same quantities of reactants. The filtrates from this repeated reaction are combined and labelled filtrate B. Filtrates A and B are combined and concentrated *in vacuo* to about 1/5 the original volume, poured into water (about 6-fold volume) and filtered. The filtercake is washed with water and dried in vacuo at 55°-60°C for 16 hours to give the title product as a pale yellow solid, 1,230.0g (85.4% yield), 98.1% pure by HPLC analysis, identified by ¹HNMR, ¹³CNMR and IR analyses.

### EXAMPLE 2

### Preparation of Ethyl 2,3-Dihydro-3,3,4-trimethylbenzo[b]thiophene-5-carboxylate, 1,1-dioxide

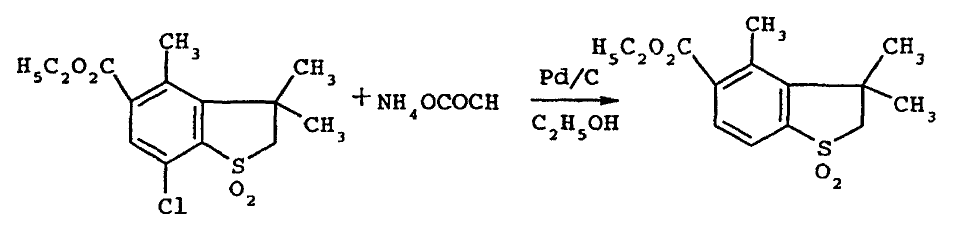

Using essentially the same procedure described in Example 1 and substituting ethyl 7-chloro-2,3-dihydro-3,3,4-trimethylbenzo[b]thiophene-5-carboxylate, 1,1-dioxide as starting material, the title product may be obtained and identified by ¹HNMR, IR and mass spectral analyses.

### EXAMPLE 3

### Preparation of 2,3-Dihydro-3,3,4-trimethylbenzo[b]-thiophene-5-carboxylic acid, 1,1-dioxide

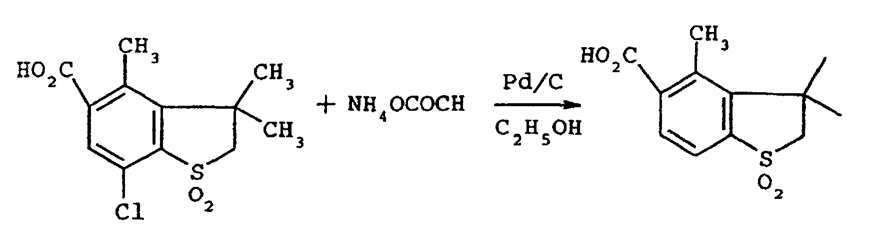

A mixture of 7-chloro-2,3-dihydro-3,3,4-trimethylbenzo[b]thiophene-5-carboxylic acid, 1,1-dioxide (1.0 g, 3.46 mmol), ammonium formate (1.0 g, 15.9 mmol) in ethanol, under nitrogen, is treated with 10% Pd/C (50% water) (0.50 g, 0.235 mmol Pd), heated at reflux temperature for 1 hour (reaction complete by HPLC analysis), cooled to room temperature and filtered through diatomaceous earth. The filtercake is washed with ethanol. The filtrates are combined and concentrated *in vacuo* to give a residue. The residue is taken up in aqueous NaOH, cooled to 5°C and acidified to pH 1 with concentrated HCl to give a precipitate. The mixture is filtered. The filtercake is washed with water and air dried to give the title product as a white solid, 0.80 g (91% yield) 99.3% pure by HPLC analysis, identified by ¹HNMR.

## Claims

1. A process for the preparation of a compound of formula (I) 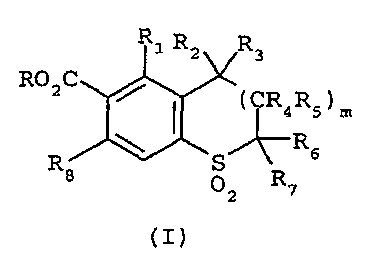 wherein
R and R₁ are each independently H or C₁-C₄alkyl;
R₂ and R₃ are each independently H, C₁-C₄alkyl or R₂ and R₃ may be taken together with the atom to which they are attached to form a group C=NOR₉ or C=O;
R₄, R₅, R₆, R₇, R₈ and R₉ are each independently H or C₁-C₄alkyl; and
m is 0 or 1
which process comprises reacting a compound of formula II 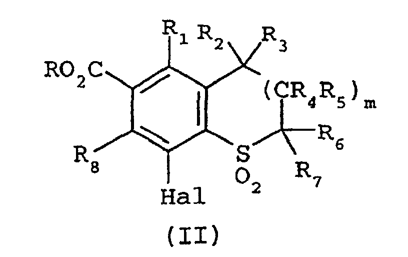 wherein Hal is Cl, Br or I and R, R₁, R₂, R₃, R₄, R₅, R₆, R₇, R₈, R₉ and m are as defined hereinabove with a catalytic amount of a transition metal catalyst and at least two molar equivalents of ammonium formate in the presence of a polar solvent optionally at an elevated temperature, and optionally hydrolyzing a formula (I) compound wherein R is C₁-C₄alkyl with base to give the corresponding carboxylic acid.

2. A process according the claim 1 wherein the solvent is a C₁-C₆alkanol, a C₁-C₆carboxylic acid, or a mixture thereof.

3. A process according to claim 1 or claim 2 wherein the transition metal is Pd.

4. A process according to any one of claims 1 to 3 wherein Hal is Cl.

5. A process according to any one of claims 1 to 4 wherein the temperature is from about 25°C to about 200°C.

6. The process according to claim 5 wherein the temperature is about 50°C-150°C

7. A process according to any one of claims 1 to 6 wherein m is 0 and R₂ and R₃ are each independently C₁-C₄alkyl.

8. A process according to any one of claims 1 to 6 wherein R₁, R₂ and R₃ are methyl; R₆, R₇ and R₈ are H; and m is 0.

9. A process according to any one of claims 1 to 6 wherein m is 1; R₂ and R₃ are taken together with the atom to which they are attached to form a group C=NOR₉; and R₉ is C₁-C₄alkyl.

10. A process according to any one of claims 1 to to 6 wherein R₁ is methyl; R₂ and R₃ are taken together with the atom to which they are attached to form a group C=NOR₉; R₉ is methyl; R₄, R₅, R₆, R₇ and R₈ are H; and m is 1.

11. A process for the preparation of a herbicidal compound of formula V or formula VII 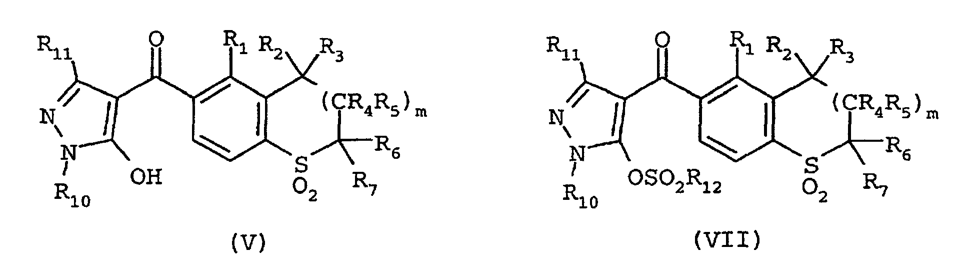 wherein
R₁, R₂, R₄, R₅, R₆, R₇, R₈, R₉ and R₁₁ are each independently H or C₁-C₄alkyl;
R₂ and R₃ are each independently H or C₁-C₄alkyl or R₂ and R₃ may be taken together with the atom to which they are attached to form a group C=NOR₉ or C=O;
R₁₀ is C₁-C₄alkyl;
R₁₂ is C₁-C₆alkyl or phenyl optionally substituted with one to three halogen, NO₂, C₁-C₄alkyl or C₁-C₄alkoxy groups; and
m is 0 or 1
which process comprises the following steps:
(a) reacting a compound of formula (II) 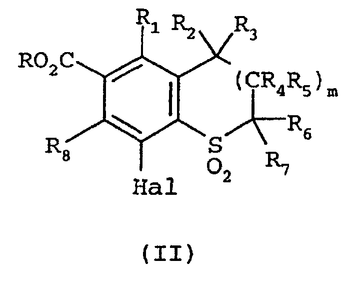 wherein Hal is Cl, Br or I; R₁₋₈ and m are as defined above and R is H or C₁₋₄alkyl with a catalytic amount of a transition metal catalyst and at least two molar equivalents of ammonium formate in the presence of a polar solvent optionally at an elevated temperature to give a corresponding compound of formula (I) 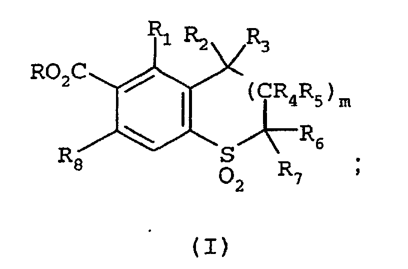 and where the product is a compound of formula (I) wherein R is C₁-C₄alkyl, hydrolysing with base to give the corresponding carboxylic acid; and
(b) reacting the product of step (a) wherein R is H with a compound of formula IV 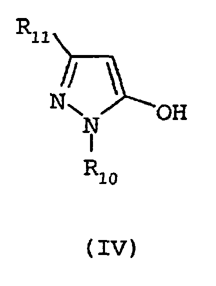 to obtain the desired herbicidal compound of formula V, optionally reacting said formula V compound with a sulfonyl chloride R₁₂SO₂Cl to obtain the desired herbicidal compound of formula VII.

12. A process according to claim 11 in which step a) is carried out according to any one of claims 2 to 10.

## Patentansprüche

1. Verfahren zur Herstellung einer Verbindung der Formel (I), 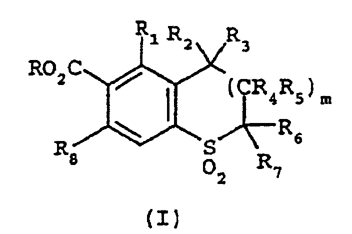 in der R und R₁ jeweils unabhängig H oder C₁-C₄Alkyl bedeuten,
R₂ und R₃ jeweils unabhängig H oder C₁-C₄Alkyl bedeuten oder R₂ und R₃ gemeinsam mit dem Atom, an das sie gebunden sind, eine Gruppe C=NOR₉ oder C=O bilden können,
R₄, R₅, R₆, R₇, R₈ und R₉ jeweils unabhängig H oder C₁-C₄Alkyl bedeuten, und
m 0 oder 1 bedeutet,
**dadurch gekennzeichnet, daß** man eine Verbindung der Formel II 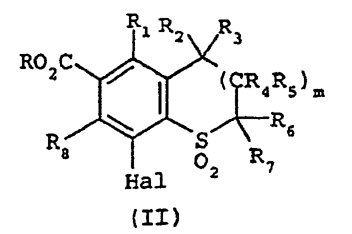 in der Hal Cl, Br oder I bedeutet und R, R₁, R₂, R₃, R₄, R₅, R₆, R₇, R₈, R₉ und m wie oben definiert sind, mit einer katalytischen Menge eines Übergangsmetallkatalysators und mindestens zwei Moläquivalenten Ammoniumformiat in Gegenwart eines polaren Lösungsmittels sowie gegebenenfalls bei erhöhter Temperatur umsetzt und gegebenenfalls eine Verbindung der Formel (I), in der R C₁-C₄Alkyl bedeutet, mit einer Base hydrolysiert, wodurch man die entsprechende Carbonsäure erhält.

2. Verfahren nach Anspruch 1, wobei es sich bei dem Lösungsmittel um ein C₁-C₆Alkanol, eine C₁-C₆Carbonsäure oder eine Mischung davon handelt.

3. Verfahren nach Anspruch 1 oder 2, wobei es sich bei dem Übergangsmetall um Pd handelt.

4. Verfahren nach einem der Ansprüche 1 bis 3, wobei Hal Cl bedeutet.

5. Verfahren nach einem der Ansprüche 1 bis 4, wobei die Temperatur ungefähr 25°C bis ungefähr 200°C beträgt.

6. Verfahren nach Anspruch 5, wobei die Temperatur ungefähr 50°C-150°C beträgt.

7. Verfahren nach einem der Ansprüche 1 bis 6, wobei m 0 bedeutet und R₂ und R₃ jeweils unabhängig C₁-C₄Alkyl bedeuten.

8. Verfahren nach einem der Ansprüche 1 bis 6, wobei R₁, R₂ und R₃ Methyl bedeuten, R₆, R₇ und R₈ H bedeuten und m 0 bedeutet.

9. Verfahren nach einem der Ansprüche 1 bis 6, wobei m 1 bedeutet, R₂ und R₃ gemeinsam mit dem Atom, an das sie gebunden sind, eine Gruppe C=NOR₉ bilden, und R₉ C₁-C₄Alkyl bedeutet.

10. Verfahren nach einem der Ansprüche 1 bis 6, wobei R₁ Methyl bedeutet, R₂ und R₃ gemeinsam mit dem Atom, an das sie gebunden sind, eine Gruppe C=NOR₉ bilden, R₉ Methyl bedeutet, R₄, R₅, R₆, R₇ und R₈ H bedeuten und m 1 bedeutet.

11. Verfahren zur Herstellung einer herbiziden Verbindung der Formel V oder der Formel VII 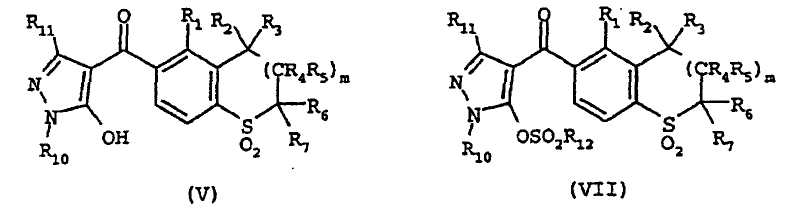 in der R₁, R₂, R₄, R_{5,} R₆, R₇, R_{8,} R₉ und R₁₁ jeweils unabhängig H oder C₁-C₄Alkyl bedeuten,
R₂ und R₃ jeweils unabhängig H oder C₁-C₄Alkyl bedeuten oder R₂ und R₃ gemeinsam mit dem Atom, an das sie gebunden sind, eine Gruppe C=NOR₉ oder C=O bilden können,
R₁₀ C₁-C₄Alkyl bedeutet,
R₁₂ C₁-C₆Alkyl oder Phenyl, das gegebenenfalls durch eine bis drei Halogen-, NO₂-, C₁-C₄Alkyl- oder C₁-C₄Alkoxygruppen substituiert ist, bedeutet, und
m 0 oder 1 bedeutet,
umfassend die folgenden Schritte:
(a) Umsetzen einer Verbindung der Formel (II) 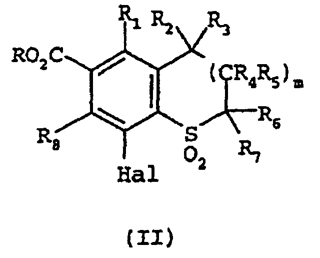 in der Hal Cl, Br oder I bedeutet, R₁₋₈ und m wie oben definiert sind und R H oder C₁-C₄Alkyl bedeutet, mit einer katalytischen Menge eines Übergangsmetallkatalysators und mindestens zwei Moläquivalenten Ammoniumformiat in Gegenwart eines polaren Lösungsmittels sowie gegebenenfalls bei erhöhter Temperatur, wodurch man eine entsprechende Verbindung der Formel (I) erhält 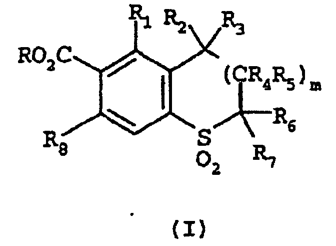 und, wenn das Produkt eine Verbindung der Formel (I) ist, in der R C₁-C₄Alkyl bedeutet, Hydrolyse mit einer Base, wodurch man die entsprechende Carbonsäure erhält, und
(b) Umsetzen des Produkts aus Schritt (a), in dem R H bedeutet, mit einer Verbindung der Formel IV 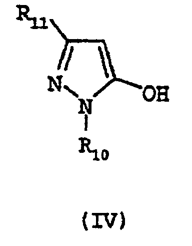 wodurch man die gewünschte herbizide Verbindung der Formel V erhält, sowie gegebenenfalls Umsetzen dieser Verbindung der Formel V mit einem Sulfonylchlorid R₁₂SO₂Cl, wodurch man die erwünschte herbizide Verbindung der Formel VII erhält.

12. Verfahren nach Anspruch 11, bei dem Schritt a) gemäß einem der Ansprüche 2 bis 10 durchgeführt wird.

## Revendications

1. Procédé pour la préparation d'un composé répondant à la formule I 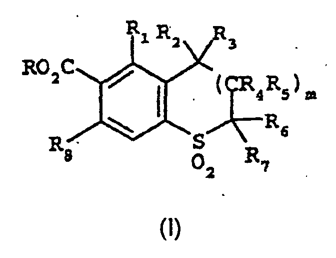 dans laquelle R et R₁ représentent chacun indépendamment un atome d'hydrogène ou un groupe alkyle en C₁-C₄ ;
R₂ et R₃ représentent chacun indépendamment un atome d'hydrogène, un groupe alkyle en C₁-C₄ ou R₂ et R₃ peuvent être pris ensemble avec l'atome auquel ils sont attachés pour former un groupe C=NOR₉ ou C=O ;
R₄, R₅, R₆, R₇, R₈ et R₉ représentent chacun indépendamment un atome d'hydrogène ou un groupe alkyle en C₁-C₄ ;
et
m vaut 0 ou 1
lequel procédé comprend la réaction d'un composé répondant à la formule II 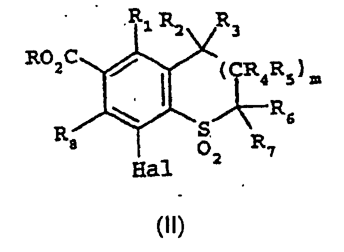 dans laquelle Hal est Cl, Br ou I et R, R₁, R₂, R₃, R₄, R₅, R₆, R₇, R₈, R₉ et m sont tels que définis ci-dessus avec une quantité catalytique d'un catalyseur de type métal de transition et au moins deux équivalents molaires de formate d'ammonium en présence d'un solvant polaire éventuellement à une température élevée, et éventuellement l'hydrolyse d'un composé répondant à la formule I dans laquelle R est un groupe alkyle en C₁-C₄ avec une base pour donner l'acide carboxylique correspondant.

2. Procédé selon la revendication 1, dans lequel le solvant est un alcanol en C₁-C₆, un acide carboxylique en C₁-C₆ ou un mélange de ceux-ci.

3. Procédé selon la revendication 1 ou 2, dans lequel le métal de transition est Pd.

4. Procédé selon l'une quelconque des revendications 1 à 3, dans lequel Hal est Cl.

5. Procédé selon l'une quelconque des revendications 1 à 4, dans lequel la température est comprise dans la plage allant d'environ 25° C à environ 200° C.

6. Procédé selon l'une quelconque des revendications 1 à 5, dans lequel la température est comprise dans la plage allant d'environ 50° C à 150° C.

7. Procédé selon l'une quelconque des revendications 1 à 6, dans lequel m vaut 0 et R₂ et R₃ représentent chacun indépendamment un groupe alkyle en C₁-C₄.

8. Procédé selon l'une quelconque des revendications 1 à 6, dans lequel R₁, R₂ et R₃ représentent un groupe méthyle ; R₆, R₇ et R₈ représentent un atome d'hydrogène ; et m vaut 0.

9. Procédé selon l'une quelconque des revendications 1 à 6, dans lequel m vaut 1 ; R₂ et R₃ sont pris ensemble avec l'atome auquel ils sont attachés pour former un groupe C=NOR₉ ; et R₉ représente un groupe alkyle en C₁-C₄.

10. Procédé selon l'une quelconque des revendications 1 à 6, dans lequel R₁ est un groupe méthyle ; R₂ et R₃ sont pris ensemble avec l'atome auquel ils sont attachés pour former un groupe C=NOR₉ ; R₉ représente un groupe méthyle ; R₄, R₅, R₆, R₇ et R₈ représentent un atome d'hydrogène ; et m vaut 1.

11. Procédé pour la préparation d'un composé herbicide répondant à la formule V ou formule VII 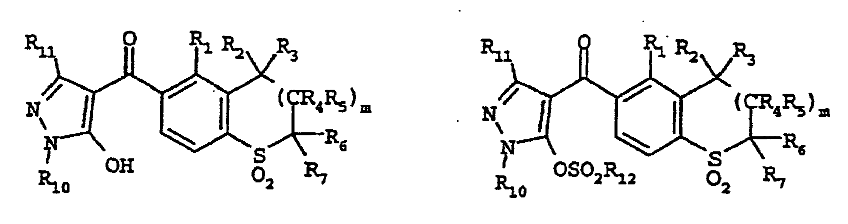 dans laquelle R₁, R₂, R₃, R₄, R₅, R₆, R₇, R₈, R₉ et R₁₁ représentent chacun indépendamment un atome d'hydrogène ou un groupe alkyle en C₁-C₄;
R₂ et R₃ représentent chacun indépendamment un atome d'hydrogène ou un groupe alkyle en C₁-C₄ ou R₂ et R₃ peuvent être pris ensemble avec l'atome auquel ils sont attachés pour former un groupe C=NOR₉ ou C=O ;
R₁₀ représente un groupe alkyle en C₁-C₄;
R₁₂ représente un groupe alkyle en C₁-C₆ ou un groupe phényle éventuellement substitué par un à trois atomes d'halogène, NO₂, groupes alkyle en C₁-C₄ ou groupes alcoxy en C₁-C₄ ; et
m vaut 0 ou 1
lequel procédé comprend les étapes suivantes consistant à :
(a) faire réagir un composé répondant à la formule II 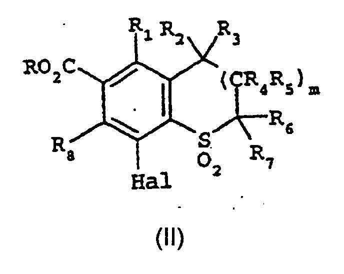 dans laquelle Hal est Cl, Br ou I, R₁₋₈ et m sont tels que définis ci-dessus et R représente un atome d'hydrogène ou un groupe alkyle en C₁-C₄ avec une quantité catalytique d'un catalyseur de type métal de transition et au moins deux équivalents molaires de formate d'ammonium en présence d'un solvant polaire éventuellement à une température élevée pour donner un composé correspondant répondant à la formule I 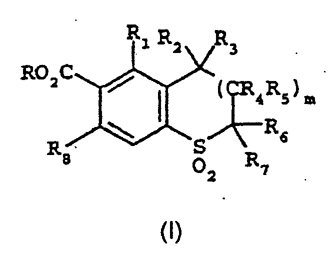 et où le produit est un composé répondant à la formule I dans laquelle R représente un groupe alkyle en C₁-C₄, hydrolysé avec une base pour donner l'acide carboxylique correspondant : et
(b) faire réagir le produit de l'étape (a) dans lequel R est un atome d'hydrogène avec un composé répondant à la formule IV 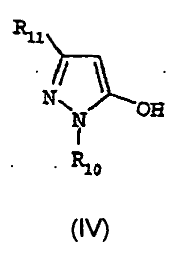 pour obtenir le composé herbicide désiré répondant à la formule V faire éventuellement réagir ledit composé répondant à la formule V avec un chlorure de sulfonyle, R₁₂SO₂Cl, pour obtenir le composé herbicide désiré répondant à la formule VII.

12. Procédé selon la revendication 11, dans lequel l'étape (a) est effectuée selon l'une quelconque des revendications 2 à 10.
